# EUROPEAN PATENT APPLICATION

(11) **EP 2 105 089 A1**
(43) Date of publication of application: **30.09.2009**
(21) Application number: 08012627.9
(22) Date of filing: 11.07.2008
(51) Int. Cl.: A61B 5/04

(54) **Electrode system for electrocardiography (ECG)**

(30) Priority: 28.03.2008 IT MI20080526
(71) Applicant: ANTEA S.r.l., 20124 Milano (IT)
(72) Inventor: Rossetti, Walter, 20144 Milano (IT)
(74) Representative: Trupiano, Federica

(57) **Abstract**

There is disclosed an electrode assembly for electrocardiogram, comprising a flexible support on which a plurality of electrodes are disposed, at least one terminal for electrical connection to an electrocardiogram instrument and a plurality of conductive tracks that connect the electrodes to the electrical connection terminal. The support advantageously includes elastically deformable portions disposed between adjacent electrodes to take the assembly from a flat configuration to a three-dimensional configuration.

## Description

The present invention relates to an electrode assembly for electrocardiogram (ECG) and, in particular, to an electrode assembly to perform a twelve-lead ECG.

To obtain an electrocardiogram it is necessary to use a special instrument, known as electrocardiograph, and a part of "external" circuit, composed of shielded connection cables and electrodes to be placed on the patient's skin. The shielded cables are necessary in order to exclude low frequency noise that would otherwise make the signal obtained illegible.

Typically, in the case of twelve-lead electrocardiogram, this is a bundle of ten cables, each with a diameter of approximately 4 mm and a maximum length of approximately 2 m. The electrodes are required to detect the signals of the polarization vector of the heart muscle and must be positioned in very precise points of the human body, in particular of the upper body, by skilled personnel.

Electrodes of known type have a standard structure which includes a "pad" of conductive material, connected to the respective cable, on which a layer of silver and a layer of silver chloride are deposited in succession. In the layers of silver chloride and silver conduction changes from ionic to electronic and, upon reaching the conductor, the signal is sent to the instrument.

Before each electrode is positioned on a patient's skin, an ion exchange contact medium in liquid state or in the form of a gel is applied to each electrode.

The structure of the electrode must be such as to prevent the "battery" effect which occurs when two different metals come into direct contact in the presence of an electrolyte, such as the contact medium with the skin. The battery effect, in this particular application, would have the disagreeable effect of saturating the electrocardiograph instrument, making it in fact impossible to detect any significant signals: the characteristic signals to be detected usually have a lower potential to the one that would be generated by two conductive metals (i.e. copper and silver) in reciprocal contact.

In order to position electrodes of known type on the patient's skin two methods are essentially used. A first method entails applying a glue for human use to an appropriate dedicated area in proximity of the electrode pad. A second method involves the use of electrodes capable of producing vacuum conditions (suction electrodes); in the latter case the contact medium is provided separately in the form of a gel to be applied to the electrode pad.

Electrodes of known type are therefore somewhat bulky and essentially intended for use in a doctor's surgery or hospital environment. Moreover, positioning of the electrodes must be performed by skilled personnel.

Following the development of telemedicine systems and of portable instruments to perform an ECG in real time, electrode assemblies that are relatively compact and easy to position, which are produced using flexible circuit technology, have recently been proposed.

An example of an electrode assembly of this type is described in the United States patent n. US 7,266,405 which presents an electrode assembly for performing a twelve-lead ECG. The assembly is produced on a flexible support which can be folded and housed in a suitable holder. When it is to be positioned on the skin, the assembly is returned to the flat condition, in which most of the electrodes, and in particular the precordial electrodes, are positioned in an inextensible portion of the flexible support.

However, arrangement of the electrodes on an inextensible support causes the introduction of an electrical noise caused essentially by the tensile stress exerted on the electrodes during breathing. The signals reaching the instrument are therefore disturbed and can falsify or even prevent correct reading of the ECG. Even in the case in which suitable filtering of the signal were to be provided to limit electrical noise of this type, part of the information detected would still be lost.

Moreover, in particular conditions of use, for example after sweating, the continuous tensile stress exerted on the electrodes can cause one or more of these electrodes to detach from the skin, thus preventing detection of all the necessary signals.

This being stated, the task of the present invention is to propose an electrode assembly for ECG that overcomes the drawbacks of the prior art.

Within the scope of this task, an object of the present invention is to propose an electrode assembly that prevents the electrical noise to occur in the signals detected.

Another object of the present invention is to propose an electrode assembly of the aforesaid type that facilitates correct positioning of the electrodes on the skin even by the patient himself.

A further object of the present invention is to propose an electrode assembly of the type specified above which can be produced in a simple and inexpensive manner.

Yet another object of the present invention is to propose an electrode assembly of the type specified above which presents a compact configuration and limited overall dimensions.

These objects are achieved by means of the present invention which relates to an electrode assembly for electrocardiogram, comprising a flexible support on which there are disposed a plurality of electrodes, at least one terminal for electrical connection to an electrocardiogram instrument and a plurality of conductive tracks that connect the electrodes to the electrical connection terminal.

The support advantageously includes elastically deformable portions disposed between adjacent electrodes to take the assembly from a flat configuration to a three-dimensional configuration.

Insertion of elastically deformable portions between the electrodes allows elimination of tensile stress exerted on adjacent electrodes caused by breathing or movement of the upper body muscles during detection of the signals by each electrode, thus preventing the electrical noises to occur in the signals detected.

According to a particular aspect of the present invention, the elastically deformable portions are produced by cuts made according to continuous lines in the flexible support. Elastically deformable connection strips between the various electrodes are thus formed in the assembly, some of which take, for example, a spiral or double spiral structure.

The cuts preferably have at least one curvilinear shaped end which extends according to a return loop toward the line of the respective cut.

With this solution it is possible to obtain a good degree of elasticity of the electrode assembly when it is positioned on the patient's skin and a high mechanical strength of the connection portions at the ends of each cut.

According to an advantageous aspect of the present invention, the flexible support incorporates a shielding in conductive material to prevent the signals detected and sent to the electrocardiograph from being affected by noise and/or external interferences.

The shielding in conductive material is structured at least partly according to a grid geometry, so as to give the support increased sturdiness, without compromising the properties of elasticity. In some portions the shielding can be solid, for example in some areas of the deformable portions, to increase mechanical strength or to convey stresses.

In a possible embodiment of the present invention, an electrically conductive adhesive material is applied to each electrode. This allows an effective signal to be obtained even without using a contact medium, such as gel or the like, further facilitating positioning of the electrodes on the skin.

Further advantages and characteristics of the present invention will be apparent from the description below, with reference to the accompanying drawings provided by way of non limiting example, in which:
- Figure 1 is a plan view of an electrode assembly according to a first embodiment of the present invention, essentially showing the position of the electrodes and the structure of the cuts;
- Figure 2A shows a portion of the electrode assembly of Figure 1 in a flat condition before use;
- Figure 2B shows a portion of the electrode assembly of Figure 2A in an extended or three-dimensional configuration during use;
- Figure 3 shows a plan view of the electrode assembly of Figure 1 in which the electrodes and the tracks to connect them to the connection terminal are highlighted;
- Figure 4 is a plan view of the electrode assembly of Figure 1 in which the configuration of the shielding in conductive material is highlighted;
- Figure 5 is a plan view of the electrode assembly of Figure 1 in which the areas in which the adhesive material is applied are highlighted;
- Figure 6A is a sectional view showing the composition of an electrode assembly according to the present invention at a connection portion;
- Figure 6B is a sectional view showing the composition of an electrode assembly according to the present invention at an electrode;
- Figure 7 schematically shows positioning of the electrodes of an assembly according to the present invention on the upper body of a patient;
- Figure 8 is a plan view of an electrode assembly according to another possible embodiment of the present invention, essentially showing the position of the electrodes and the structure of the cuts;
- Figure 9 is a plan view of the electrode assembly of Figure 8 in which the electrodes and the tracks for connection thereof to the connection terminal are highlighted;
- Figure 10 is a plan view of the electrode assembly of Figure 8 in which the configuration of the shielding in conductive material is highlighted; and
- Figure 11 is a plan view of the electrode assembly of Figure 8 in which the areas in which the adhesive material is applied are highlighted.

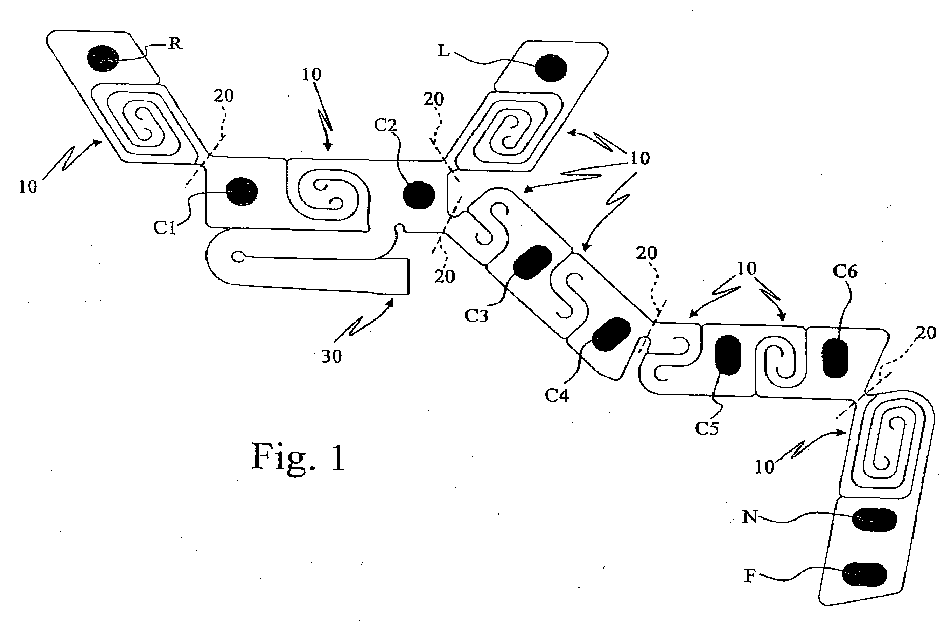

The view of Figure 1 represents an electrode assembly according to a first embodiment of the present invention, to perform a twelve-lead electrocardiogram. The assembly comprises six "internal" (or precordial) electrodes indicated by the references C1-C6 and "external" electrodes indicated by the references R, L, N and F, each of the electrodes having a circular or oval shape.

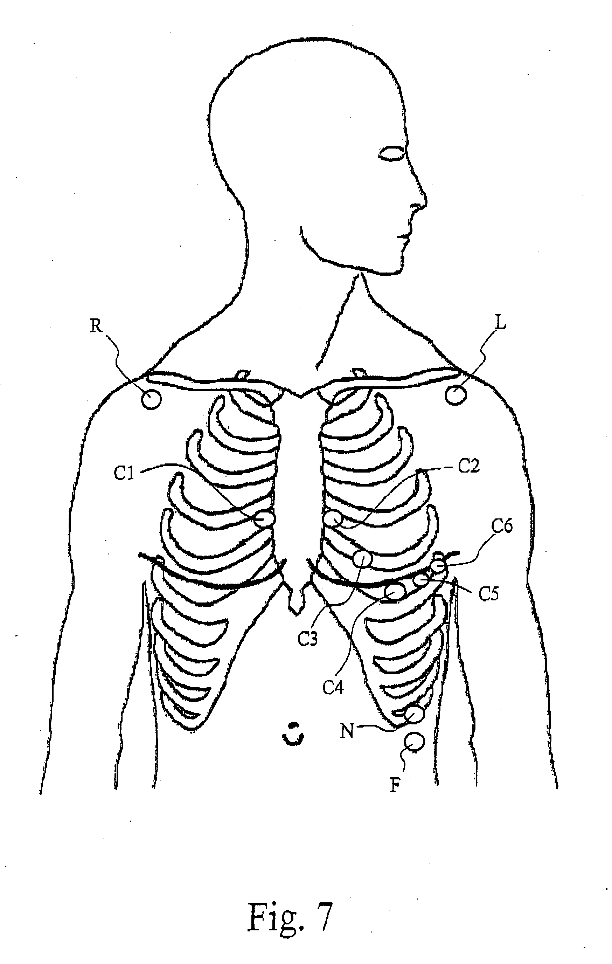

Table 1 below indicates the correspondence between the nomenclature of the electrodes in Figure 1 and the definition of their position, which will be shown in greater detail in Figure 7.

**Table 1**

| ***Electrode*** | ***Definition*** |
|---|---|
| L | left wrist |
| R | right wrist |
| C1 | fourth intercostal space at the right sternal edge |
| C2 | fourth intercostal space at the left sternal edge |
| C3 | midway between C2 and C4 |
| C4 | fifth intercostal space on the mid-clavicular line |
| C5 | midway between C4 and C6 |
| C6 | left mid-axillary line at the same level as C4 |
| N | right ankle |
| | (reference electrode) |
| F | left ankle |

The electrodes are disposed on a flexible support made of plastic material which includes a plurality of elastically deformable portions 10 that allow the assembly to be taken from a flat configuration, ideal for packaging the electrode assembly before use, to a three-dimensional configuration that allows correct positioning on the patient.

The view of Figure 1 also highlights the fold lines 20 along which the various parts of the electrode assembly can be folded so that it can be packaged or stored with particularly limited overall dimensions.

Therefore, this geometry of the electrode assembly allows the final overall dimensions of the assembly packaged before use to be particularly limited and intuitively facilitates positioning thereof.

In particular, the electrodes C1 and C2 are located at the same height, the electrodes C2, C3 and C4 are mutually aligned along a directrix inclined with respect to the horizontal by approximately 45°, the electrodes C4, C5 and C6 are mutually aligned and at the same height. Moreover, the precordial electrodes are disposed two by two along three main directrices and these latter are mutually connected at two folding axes, so as to allow folding of the assembly along a single directrix.

A further contribution to reducing the area occupied by the assembly is also given by the arrangement of the external electrodes. For example, the reference electrode N is disposed in proximity of the electrode F on a same portion of the flexible support and the electrodes R and L are connected respectively to the electrodes C1 and C2 by elastically deformable portions 10. Finally, the connector 30 is disposed in proximity of the electrode C2, i.e. in an area easily accessible by the patient, who can thus easily position the electrode assembly without necessarily requiring the assistance of skilled personnel.

Connection between the external electrodes and the precordial electrodes is also obtained through elastically deformable portions 10, which allow the external electrodes to be maintained, in flat condition, close to the precordial electrodes, but to be moved away from these when they are positioned on the body.

The sections of the assembly comprising the external electrodes are positioned along three directrices that intuitively provide indications for correct positioning of the electrodes. These three directrices have been connected to the precordial electrodes at three folding axes, so that they too can be folded over the shapes of the precordial electrodes. By way of example, a prototype of the assembly was produced at natural size, which in the flat (in the condition shown in Figure 1) was inscribed in a rectangle of approximately 276 x 205 mm, but once folded along all the folding lines 20 its dimensions were reduced to those of a rectangle of approximately 90 x 29 mm.

Again with reference to Figure 1, the geometry of the electrode assembly according to this first embodiment was designed to allow the assembly to be folded following the steps indicated below:
- folding of the electrode R over the directrix C 1-C2;
- folding of the electrode L over the directrix C1-C2;
- folding of the electrode F over the directrix C5-C6;
- folding of the directrix C5-C6 over the directrix C3-C4;
- folding of the directrix C3-C4 over the directrix C1-C2; and
- folding of the branch of the connector over the directrix C1-C2;

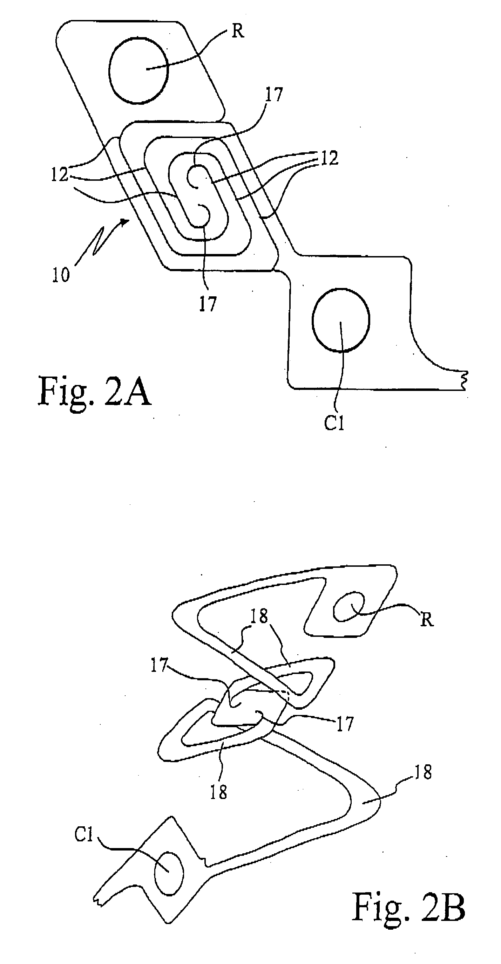

Figure 2A represents a part of the electrode assembly of Figure 1 in flat condition, in particular the part comprising the two electrodes R, C1 and an elastically deformable portion 10 comprised therebetween.

Each elastically deformable portion 10 is produced by cuts 12 made according to continuous lines for the entire section of the assembly. At least one end of the cuts 12 terminates according to a curvilinear configuration that extends according to a return loop 17 toward the line of the respective cut.

In this way, during positioning, the elastically deformable portion 10 allows strips 18 with a double spiral structure to be obtained, as shown in Figure 2B, which represents the same part of the assembly as Figure 2A but extended in three-dimensional condition.

Elastically deformable portions 10 in the form of strips are also used for the other electrodes not represented in Figures 2A and 2B, thus allowing conductive tracks with increased continuity to be produced.

With the solution adopted by the present invention it is possible to contain the plan extension and improve the "fit" of the electrode assembly.

It has been found that a curvilinear configuration for the ends 17 of the cuts 12 in particular allows the assembly to be given greater mechanical resistance to tensile stresses with respect to other possible open or also closed linear configurations, such as holes made at the ends. In practice, the incision paths curved at the ends 17 allow tensile stresses to be concentrated in particularly strong areas.

It has in fact been found that a return loop 17 at the ends of the cuts allows maximization of the diameter of a "fictitious" hole on which the stresses caused by deformation of the circuit are discharged. This solution allows a certain continuity to be maintained in the material and makes further machining (i.e. perforation) in the production step unnecessary. All in all, the shape of the precordial electrodes remains particularly soft and comfortable when positioned, so as to allow use also during athletics and/or professional sporting activities.

The cuts can be produced using die cutting techniques or also by laser beam cutting.

The structure of the spiral (or coiled) strips 18 allows a particularly compact circuit to be designed, which can undergo elongations such as to allow the definition of a single model which adapts to various body sizes. In fact, the relative variations in the distances between precordial electrodes from patient to patient are limited to the order of a few centimetres.

The electrode assembly of the present invention is positioned on the skin according to a procedure of subsequent steps, so that the patient does not require to position more than two electrodes at a time on the skin: the shaped geometry facilitates this operation providing a guide for positioning the electrodes in the correct areas. The procedure provides for initial positioning of the precordial electrodes, starting from the electrode C1, then continuing with the other precordial electrodes C2-C6 and terminating with the external electrodes R, L, N and F.

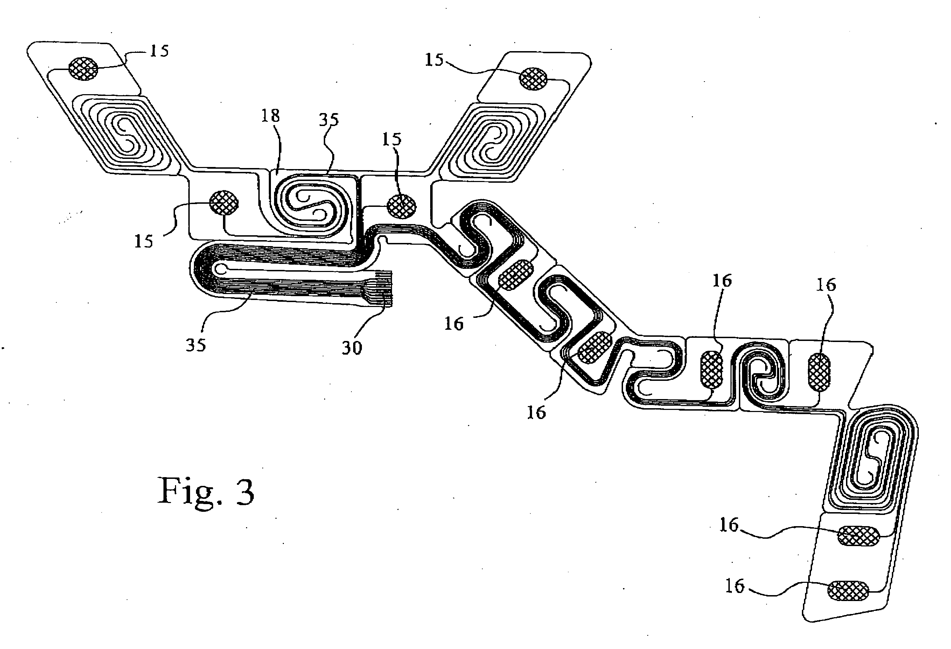

Figure 3 represents the arrangement of the conductive tracks 35 connecting the conductive pads of circular shape 15 and those of oval shape 16 (positioned at the electrodes) to a terminal 30 for electrical connection of the electrode assembly directly to an ECG instrument, or to a device to transmit the signals to a remote receiving unit, such as a mobile telephone or a suitably arranged ECG instrument. One or more conductive tracks 35 can be disposed on each of the strips 18, such as the two tracks 35 on the strip 18 identified in Figure 3.

The width of each strip 18 will be determined not only by the compromise between the contrasting requirements of minimum overall dimensions and sufficient mechanical strength, but also by the number of conductive tracks 35 disposed on each strip, bearing in mind that the tracks must have adequate dimensions to ensure high quality transmission of the signal detected and must be spaced at sufficient distance from one another on a same strip to facilitate their production and in any case to prevent interferences from being generated. For example, it has been found that the width of the tracks can be between 0.3 and 0.6 mm, with a distance between centres of the tracks that can vary from 0.6 to 1 mm.

The connection terminal represented here by way of example is produced by a connector of the ZIFF type normally used to connect flexible circuits to other circuits or devices. It is however possible also to use other types of connectors, such as rigid connectors to be welded on the assembly.

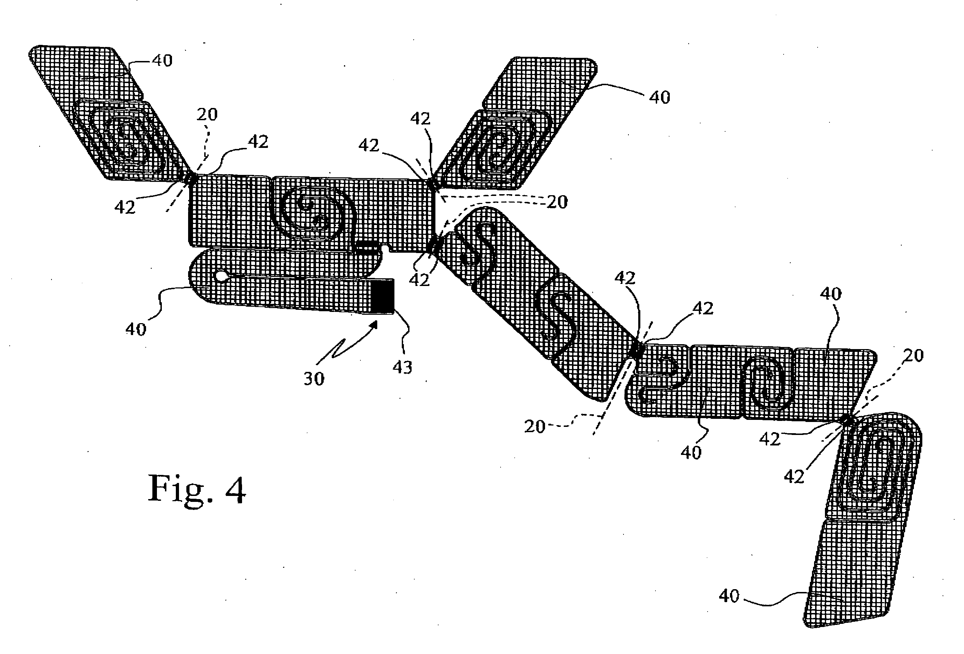

The view of Figure 4 highlights the configuration of the shielding 40 in conductive material which is disposed on the opposite side of the conductive tracks and electrically insulated therefrom.

The shielding 40 is prevalently structured according to a grid geometry, for example with square mesh such as that represented in Figure 4. Besides effectively protecting the conductive tracks and the electrodes from external interferences, this geometry offers high mechanical strength to the electrode assembly without compromising the flexibility thereof.

In particular, the shielding 40 is particularly effective against noise linked to the frequency of 50 Hz (normally present when standard cables are used), thereby making it possible to obtain a significant decrease in the noise at the electrocardiograph input and consequently traces substantially free from noise that would otherwise require to be filtered.

Along the folding lines 20 the shielding 40 can comprise solid reinforcing portions 42 disposed in pairs at the side of each line 20 to ensure correct folding of the assembly with respect to the lines. A portion of solid shielding 43 is also provided at the connection terminal 30 to provide an adequate ground contact.

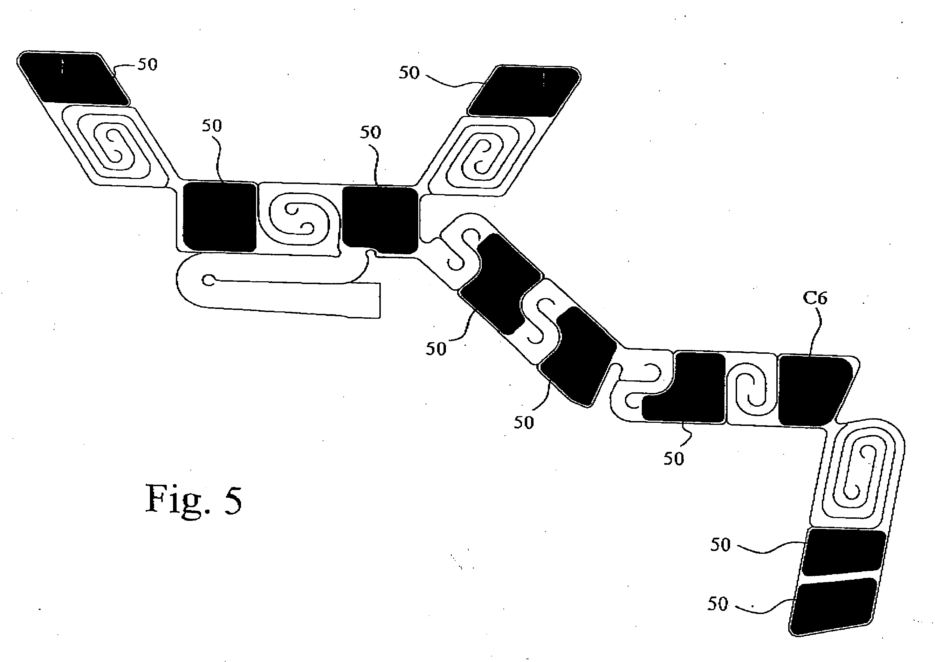

Finally, Figure 5 shows the arrangement of the adhesive material on the electrode assembly of the embodiment shown in Figures 1 to 4. In this case, it is an electrically conductive adhesive material which can be applied directly to the electrodes and to the entire surface surrounding the electrodes according to configurations indicated with the numeric reference 50. In the packaging of the electrode assembly a removable protective film (not shown) is also provided disposed on top of the adhesive layer.

With respect to the prior art of ECG electrodes, the conventional contact medium can be replaced by a contact medium simultaneously presenting properties of adhesion and electrical conductivity. In tests performed with solid adhesive and conductive gel, high level electrocardiograph traces were obtained, all without noise at the frequency of 50 Hz. Moreover, by using a conductive adhesive material as contact medium, this allows greater freedom in the choice of the shape of each electrode, providing it has an adequate surface area to detect an effective signal.

It was found that the use of a similar adhesive and conductive medium also allows a decrease in the area of silver chloride required to produce an electrode. In fact, while with the known contact medium it was necessary to produce an area of silver chloride equivalent to the area occupied by the contact medium on the support, with the use of an adhesive contact medium the area of silver chloride can be limited to the pad area of the electrode, with a decrease of approximately 75% in the material used.

It is evident that, in the event of a non conductive adhesive material being used, the adhesive layer would only require to surround the electrodes, which would require to be covered with a suitable contact medium, for example in the form of gel or the like, before being positioned on the patient. The contact medium can in any case be supplied separately or deposited in advance on the electrodes of the assembly, in this case providing a covering film to be removed prior to positioning thereof.

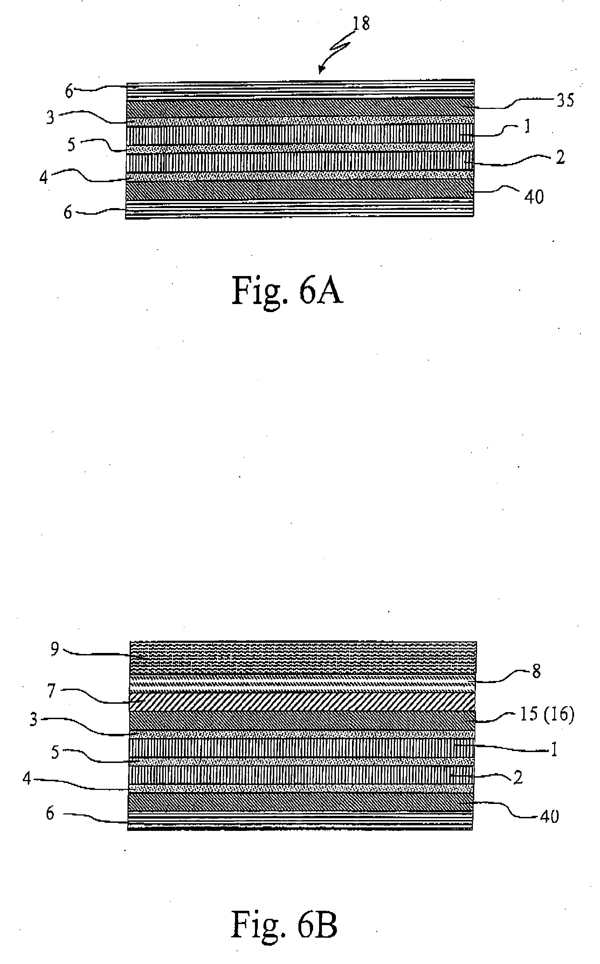

The view in Figure 6A represents a section (not in scale) showing the composition of an electrode assembly according to the present invention at a strip 18 (Figure 2B) of a connection portion.

The flexible support is composed of two layers of flexible material, indicated by the numeric references 1 and 2, which are made of plastic material, such as polyester or Kapton®, with thickness varying from 17 to 50 microns.

A layer of adhesive material 3 is applied to the plastic layer 1 to allow a layer of conductive material, such as copper, to be applied to form the conductive tracks 35 and the pads 15 and 16 to produce the electrodes. In the same way, a layer of adhesive material 4 is applied to the layer 2, which allows a second layer of conductive material, also copper, to be applied to form the shielding 40.

Formation of the tracks 35 and of the respective pads 15 and 16 at the electrodes, and formation of the geometry chosen for the shielding 40, are obtained, for example, using photoetching processes, operating on layers of copper with a thickness between 17 and 35 microns.

Once the conductive tracks 35, the pads for the electrodes 15 and 16, and the shielding 40 have been formed the two layers 1 and 2 are joined together by means of a layer of adhesive material 5 so as to maintain the tracks and the conductive pads on the opposite side to the shielding of the assembly thus produced.

The conductive layers 35 and 40 are then covered with a respective protective layer 6, made of electrically insulating material, each produced for example in the form of a film or of a paint applied to the conductive layers. The thickness of each protective layer can vary between 20 and 100 microns. With respect to the embodiment represented here, several layers of electrically insulating material can also be provided to increase the mechanical strength and allow more reliable folding.

The choice of thicknesses of the various layers of material can depend on various factors. For example, a total thickness that is too high can increase the rigidity of the assembly and cause some electrodes to detach after positioning thereof, while a total thickness that is too low can reduce the mechanical strength of the assembly during application.

The decision to produce the electrode assembly according to the invention adopting the technology of flexible electronic circuits therefore allows the conductors, pads and shielding to be produced on a flexible support. In the schematic section of Figure 6A it can be observed that, in the embodiment of the electrode assembly, two flexible circuits have substantially been coupled, a first circuit to produce the conductive tracks and the electrodes, and a second circuit to produce the ground plane to shield the tracks.

The sectional view of Figure 6B (not in scale) instead represents the composition of an electrode assembly according to the present invention at an electrode.

The composition below each electrode is the same as that shown in Figure 6A, with the exception of the upper protective layer 6. A pad 15 or 16 having the same shape and the same plan dimensions as the electrode to be produced is formed on the copper conductive layer connected to a respective conductive track. A layer of silver 7 is deposited on the pad and a layer 8 of silver chloride is in turn deposited thereon.

The reference 9 indicates an upper layer of material that is placed on top of the layer 8 of silver chloride. The layer 9 can, for example, represent a contact medium in the form of gel, in which case the layer of adhesive material for positioning on the patient's skin is not visible as it surrounds the electrode, or an electrically conductive adhesive applied to the electrodes similarly to the representation in Figure 5.

The embodiment shown in Figures 1 to 5 has a structure such as to intuitively facilitate positioning of the electrode assembly on the patient.

In fact, Figure 7 shows the positions in which the electrodes are positioned on the patient according to the nomenclature adopted in Table 1 and in Figure 1.

Positioning can start, for example, from the internal electrodes C1 and C2 which are placed at the fourth intercostal space respectively at the right and left sternal edges. Once these first two electrodes have been positioned, the subsequent internal electrodes C3-C6 are placed in the corresponding positions indicated in Table 1.

The external electrodes are then positioned, for example starting from the electrode R (extending the assembly as shown by way of example in Figure 2B) followed by the electrode L. These two electrodes, which represent the signals detected respectively at the right wrist and at the left wrist, are positioned at the height of the patient's shoulders.

Finally, the remaining external electrodes N and F are positioned. Of these two, which generally correspond to the signals detected at the ankles, the electrode N (right ankle) is used as reference electrode, while the electrode F (left ankle) is placed at the height of the navel, moved towards the left side, in proximity of the electrode N.

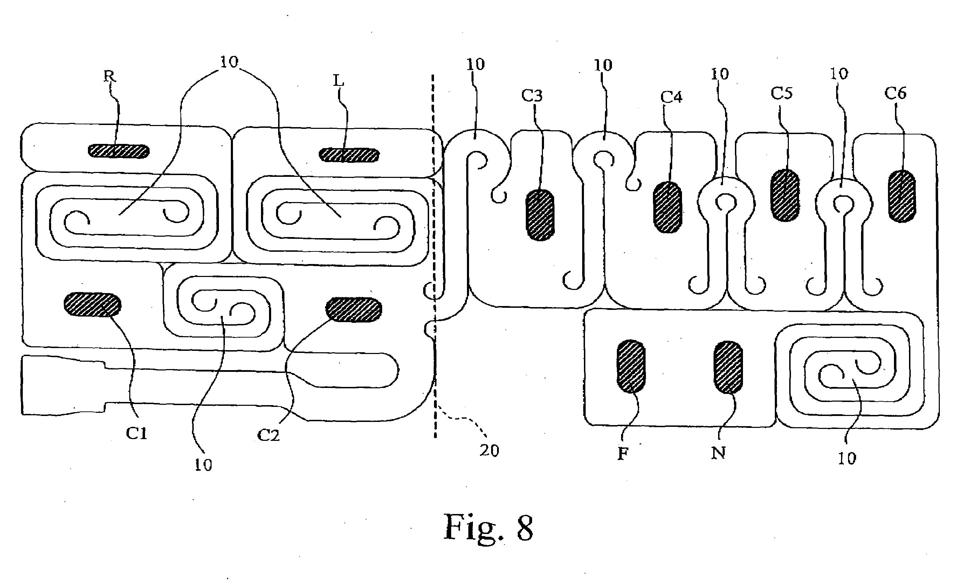

Figures 8 to 11 show a second embodiment of the present invention through which the assembly of sensors can be produced in a more compact and inexpensive form, above all due to the flat shape inscribed within a substantially rectangular contour of reduced dimensions which allows waste to be minimized and therefore a more rational use of materials. The elements common to the previous embodiment are indicated by the same references.

The view of Figure 8 thus represents an electrode assembly for performing a twelve-lead electrocardiogram, with six "internal" electrodes C1-C6 and four "external" electrodes R, L, N and F.

Also in this case the flexible support includes a plurality of elastically deformable portions 10 which allow the assembly to be taken from a flat configuration to a three-dimensional configuration. In this embodiment, a single folding line 20 is highlighted, which allows the electrode assembly to be folded and to be packaged or put away with particularly limited overall dimensions.

A prototype was produced on the basis of this second embodiment, which in flat configuration (shown in Figure 8) had overall dimensions inscribed in a rectangle of approximately 188 x 66 mm, while in folded condition the dimensions were reduced to approximately 110 x 66 mm.

The elastically deformable portions 10 have the same structure as those already described for the previous embodiment, i.e. with cuts made according to continuous lines for the entire section of the assembly and which present, at one end thereof a curvilinear configuration that extends according to a return loop toward the line of the respective cut. The effect obtained during positioning on a patient is therefore the same as that represented in Figure 2B with reference to the first embodiment. In the same way, the geometry of this second embodiment allows all the sites for positioning of the electrodes already shown for the first embodiment to be reached.

With respect to the first embodiment, the "rectangular" structure of this second embodiment causes modification of the connection sections between the precordial electrodes from C3 to C6, which have been composed of more compact connection portions than the previous ones. For example, the elastically deformable portions 10 between the electrodes C5 and C6 are produced by more compact transverse "coils" which are inserted in lower area of the supporting parts of the electrodes: in this way extension of the spirals or coils is maximized, minimizing the space required when the assembly is in flat, or inactive, condition. The elastically deformable portions 10 between the electrodes are in any case always designed to facilitate the procedure to position the assembly.

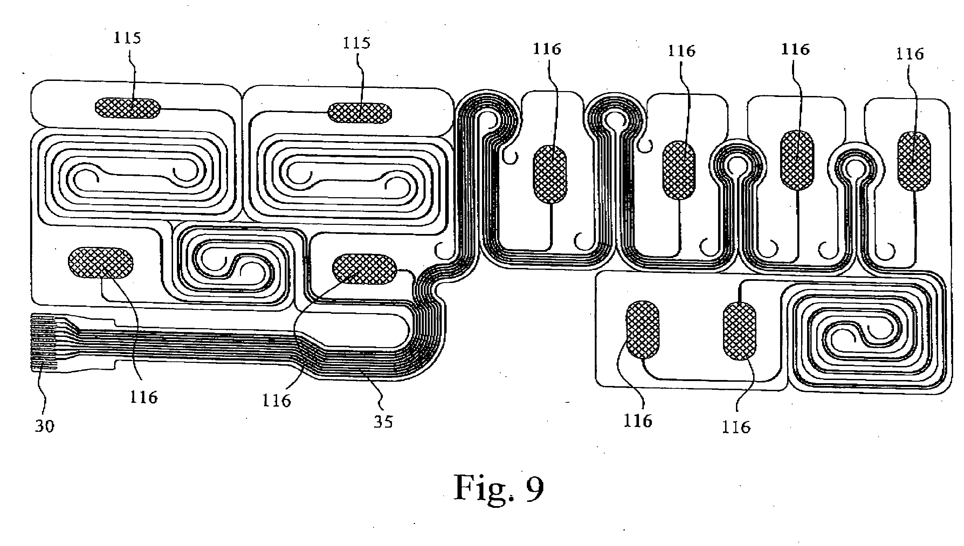

Figure 9 represents the arrangement of the conductive tracks 35 which connect the pads 115 and 116 to the terminal 30 for electrical connection of the electrode assembly, directly or indirectly, to an ECG instrument.

The pads 115 disposed at the electrodes R and L have in this case a similar shape to that of the remaining pads 116 of the remaining electrodes, but a slightly smaller surface area. However, any other shape can be adopted for each pad or electrode, providing it has a surface area suitable to ensure acquisition of a clean signal of sufficient intensity.

The grid representation of the pads mirrors the effective production thereof: it is thus possible to obtain a more flexible and "softer" structure of the electrode sensor. The grid structure can be adopted not only for the layer of copper but also for the layer of silver and also for the layer of silver chloride, if necessary.

In fact, it has been found that below a certain surface area the electrodes supply a signal affected by high electrical noise. For example, it has been found that circular pads must have a diameter of at least 4 mm in order to avoid as much as possible the noise to occur.

Also for the embodiment represented here, the conductive tracks 35 have a width between 0.3 and 0.6 mm, with distance between centres of the tracks that can vary between 0.6 and 1 mm.

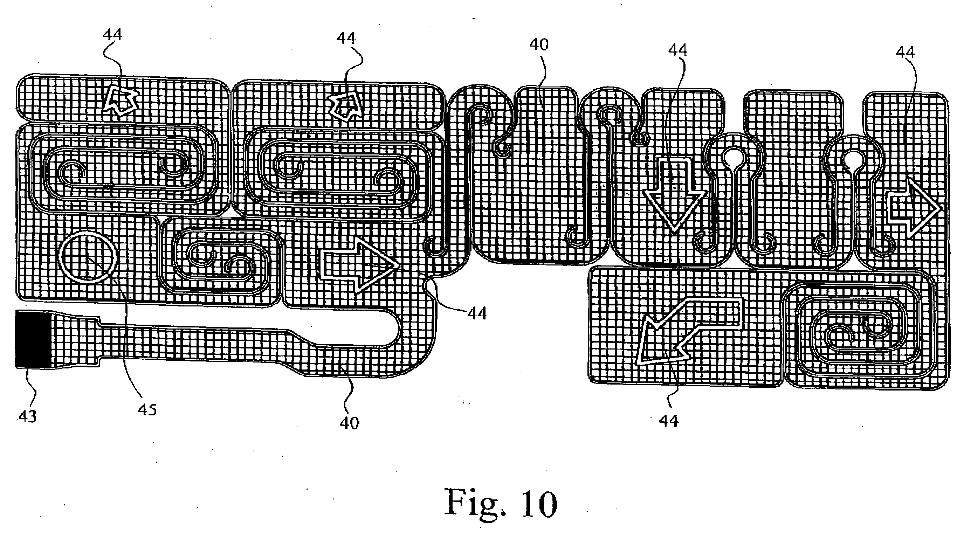

Figure 10 highlights the configuration of the shielding 40 in conductive material which is disposed on the opposite side of the conductive tracks and electrically insulated therefrom.

Similarly to the previous embodiment, the shielding 40 is prevalently structured according to a grid geometry with square mesh. A portion of solid shielding 43 is provided at least at the connection terminal 30 to provide adequate ground contact.

In order to facilitate positioning of the electrodes on the patient, symbols 44 and 45 are advantageously provided on the shielding grid 40, to indicate the directions of extension of the parts of the electrode assembly to take it from a flat to a three-dimensional configuration. For example, the first electrode to be positioned can be the one indicated by a symbol 45 in the form of a circle (corresponding to the position of the internal electrode C1) and the subsequent electrodes can be positioned after having extended the assembly along the directions indicated by the arrow symbols 44.

In particular, the stepped positioning procedure is performed in the same way as already described for the previous embodiment, i.e. so that no more than two electrodes require to be positioned at one time. The starting point is given by positioning of the precordial electrodes C1 and C2, then the external arm electrodes R and L must be positioned, followed by the remaining precordial electrodes C3-C6 and terminating with the leg electrode F together with the reference electrode N.

Simplification of the geometry in any case enables the patient to be entrusted with understanding the instruction, which is already inherent in the shape of the previous embodiment. In fact, even if the external electrodes are no longer at the ends of the inclined directrices typical of the first embodiment, the symbols 44 and 45 provide the patient with the necessary indications to position the various electrodes correctly, for example at the shoulders and at the side of the navel.

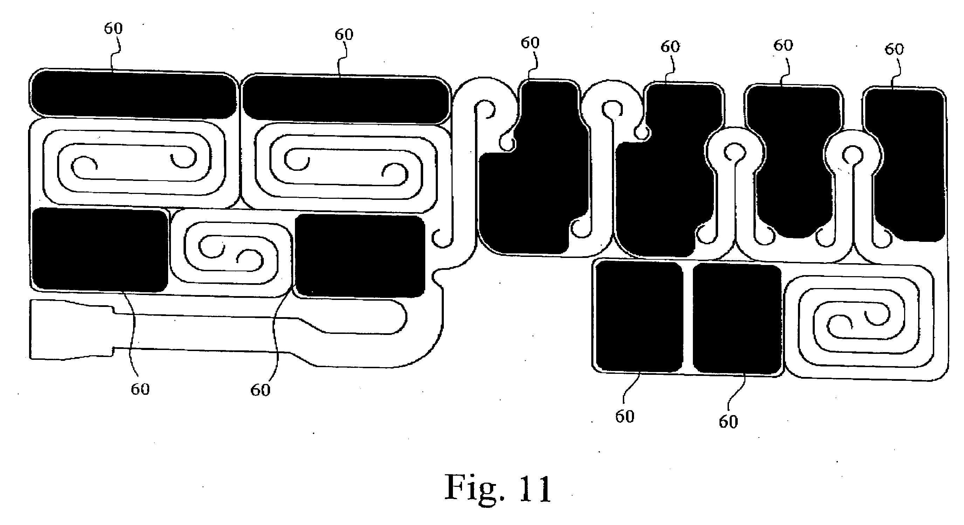

Finally, Figure 11 shows the arrangement of the adhesive material on the electrode assembly of the embodiment shown in Figures 8 to 10. This case also provides for the application of an electrically conductive adhesive material, which can thus be applied directly to the electrodes and over the entire surface surrounding these electrodes according to configurations indicated with the numeric reference 60.

The composition of the electrode assembly of this second embodiment (Figures 8-11) corresponds to that already described with reference to Figure 6A and 6B for the first embodiment.

The electrode assembly according to the present invention is suitable to produce disposable bands of sensors intended both for direct connection to an electrocardiograph instrument or for a portable transmission device to allow the signals detected to be sent to a remote unit for analysing the ECG.

Various modifications can be made to the embodiments represented here without departing from the scope of the present invention. For example, to obtain the conductive tracks, the pads and the shielding, techniques can be used which provide for printing of the conductive tracks and of the pads with inks containing silver, while formation of the shielding can also be obtained through deposition of a layer of graphite on the opposite side of the same layer of plastic support.

Moreover, the electrodes can be connected to one another according to a different structure than the one represented in the two embodiments represented herein, for example with the electrodes R and L both connected to the electrode C2, or with the electrodes F and N connected to the electrodes C2 or C4 instead of the electrode C6.

Moreover, the assembly of Figure 8 produced according to a flat rectangular geometry can be foldable according to two or more folding lines, instead of the single line 20 effectively represented, to further reduce the dimensions of the packaged assembly until substantially taking the dimensions of a "credit card", if necessary also changing the type of connection strip between the various electrodes.

Finally, although the present invention has been described with reference to an assembly of twelve electrodes, it is evident that the same production principles can be applied to assemblies having a different number of electrodes, for example assemblies having three or six electrodes.

## Claims

1. An electrode assembly for electrocardiogram, comprising a flexible support on which a plurality of electrodes are disposed, at least one terminal for electrical connection to an electrocardiogram instrument and a plurality of conductive tracks that connect said electrodes to said electrical connection terminal, **characterized in that** said support includes elastically deformable portions disposed between adjacent electrodes to take the assembly from a flat configuration to a three-dimensional configuration.

2. The electrode assembly as claimed in claim 1, wherein said elastically deformable portions are produced by cuts made according to continuous lines in the entire section of the assembly.

3. The electrode assembly as claimed in claim 2, wherein said cuts have at least one curvilinear shaped end which extends according to a return loop toward the line of the respective cut.

4. The electrode assembly as claimed in claim 1, wherein said flexible support incorporates a shielding in conductive material.

5. The electrode assembly as claimed in claim 4, wherein said shielding in conductive material is at least partly structured according to a grid geometry.

6. The electrode assembly as claimed in claim 1, wherein at least said conductive tracks are covered with at least one protective layer of insulating material.

7. The electrode assembly as claimed in claim 1, wherein an adhesive material is applied in proximity of said electrodes.

8. The electrode assembly as claimed in claim 1, wherein an electrically conductive adhesive material is applied to said electrodes.

9. The electrode assembly as claimed in claim 1, wherein said flexible support includes one or more layers of polyester.

10. The electrode assembly as claimed in claim 1, wherein said flexible support includes one or more layers of Kapton®.

11. A band of sensors for an electrocardiogram instrument, **characterized by** comprising an electrode assembly as claimed in any one of claims 1 to 10.

12. A band of sensors for an electrocardiogram instrument of portable type, **characterized by** comprising an electrode assembly as claimed in any one of claims 1 to 10.

13. A disposable band of sensors for an electrocardiogram instrument, **characterized by** comprising an electrode assembly as claimed in any one of claims 1 to 10.

14. The band of sensors according to any one of claims 11 to 13, wherein said electrode assembly is folded around at least one folding line.
